# EUROPÄISCHE PATENTSCHRIFT

(11) **EP 3 908 127 B1**
(45) Veröffentlichungstag und Bekanntmachung des Hinweises auf die Patenterteilung: **01.07.2026**
(21) Anmeldenummer: 19700865.9
(22) Anmeldetag: 09.01.2019
(51) Int. Cl.: A23L 33/15, A23L 35/00, A61K 8/92, A61K 8/9767, A61K 8/9794, A23L 29/10, A23L 33/115, A61Q 7/00, A23L 33/105

(54) **ZWEIPHASIGES SYSTEM**
TWO-PHASE SYSTEM
SYSTÈME DIPHASIQUE

(43) Veröffentlichungstag der Anmeldung: 17.11.2021
(73) Patentinhaber: PM-International AG, 5445 Schengen (LU)
(72) Erfinder: SORG, Rolf, 5444 Schengen (LU); MESSER, Wilhelm, 67098 Bad Dürkheim (DE); LAUINGER, Christian, 76275 Ettingen (DE); MEINHARDT, Horst, 56249 Herschbach (DE); HERZ, Karina, 54294 Trier (DE)
(74) Vertreter: Meissner Bolte Partnerschaft mbB
(86) Internationale Anmeldenummer: PCT/EP2019/050402
(87) Internationale Veröffentlichungsnummer: WO 2020/143910

(56) Entgegenhaltungen:
- DE-A1- 102010 007 736
- US-A1- 2010 247 563
- US-A1- 2018 092 832
- MINTEL GROUP LTD: "Shampoo", GNPD, MINTEL, 1 February 2011 (2011-02-01), XP002793860
- MINTEL GROUP LTD: "Platinum Stem Cell Elixir", GNPD, MINTEL, 1 April 2018 (2018-04-01), XP002793861
- MINTEL GROUP LTD: "Shampoo - Anti-Hair-Loss", GNPD, MINTEL, 1 August 2016 (2016-08-01), XP002793862
- DATABASE GNPD [online] MINTEL; 1 February 2011 (2011-02-01), MINTEL GROUP LTD.: "Shampoo", XP002793860, Database accession no. 1484751
- DATABASE GNPD [online] MINTEL; 1 April 2018 (2018-04-01), MINTEL GROUP LTD.: "Platinum Stem Cell Elixir", XP002793861, Database accession no. 5599791
- DATABASE GNPD [online] MINTEL; 1 August 2016 (2016-08-01), MINTEL GROUP LTD.: "Shampoo - Anti-Hair-Loss", XP002793862, Database accession no. 4229859
- KLIGMAN A M ET AL: "History of baldness from magic to medicine", CLINICS IN DERMATOLOGY, J.B. LIPPINCOTT, PHILADELPHIA, PA, US, vol. 6, no. 4, 1 October 1988 (1988-10-01), pages 83 - 88, XP026188296, ISSN: 0738-081X, [retrieved on 19881001], DOI: 10.1016/0738-081X(88)90070-3

## Beschreibung

Die Erfindung betrifft ein zweiphasiges System und ein Kit (Set), umfassend ein (erfindungsgemäßes) zweiphasiges System zur topischen Applikation auf der Kopfhaut und ein (erfindungsgemäßes) zweiphasiges System zur oralen Applikation. Die Erfindung betrifft auch die Verwendung eines erfindungsgemäßen zweiphasigen Systems oder eines erfindungsgemäßen Kits, insbesondere zur Stärkung des Haarwuchses. Ferner bezieht sich die Erfindung auf ein Verfahren zur Herstellung eines erfindungsgemäßen zweiphasigen Systems.

### Hintergrund der Erfindung

Haarausfall stellt heutzutage ein Problem für eine bedeutende Anzahl von Männern und Frauen dar. Besonders bei Männern ist die androgenetische Alopezie verbreitet. Frauen sind dagegen häufiger von telogenem Effluvium betroffen, einem diffusen Haarverlust verursacht durch Hormonschwankungen, Stress, Schwangerschaft, Operationen, Medikamente und Mangelernährung. Zur Bekämpfung des Haarausfalles werden einige potente Wirkstoffe eingesetzt, zum Beispiel Minoxidil und Finasterid. Deren Einsatz ist allerdings auf bestimmte Indikationen beschränkt und diese Wirkstoffe weisen Nebenwirkungen auf. Es besteht nach wie vor ein Bedarf für Produkte, die dem Haarausfall entgegenwirken, arm an Nebenwirkungen sind und natürliche Inhalts- und Wirkstoffe aufweisen.

Aus dem Stand der Technik sind verschiedene Zusammensetzungen bekannt, die zur Haut- und Haarpflege verwendet werden können.

A M Kligman et al veröffentlichten schon 1988 zu dem Thema Haarausfall (Clin Dermatol. 1988 Oct-Dec;6(4):83-8. doi: 10.1016/0738-081x(88)90070-3. History of baldness. From magic to medicine).

Das Shampoo Style Aromatherapy Hydro Botanic Series von E.L. Erman Cosmetic Manufacturing verfügt über eine Formel, die mit Mineralien aus dem Toten Meer, der Style Natural Anti-Ageing Technology und einem Meeresalgenkomplex aus Meeresalgen, Leinsamen, Borretschsamen und Immortelleextrakt angereichert ist.

Das Anti-Haarausfall-Shampoo Melica Organic Shampun' proty Vypadinnya Volossya wurde von Melisa Laboratorium entwickelt, um das Haar zu stärken und sein Wachstum zu fördern. Die Formel enthält 95 % Inhaltsstoffe natürlichen Ursprungs und stärkende Extrakte aus: Roggen, der reich an Vitaminen sowie Mikro- und Makroelementen ist, um die Haarfollikel zu nähren und Haarausfall zu reduzieren; Kalmus, um die Aktivität der Haarfollikel anzuregen und den Feuchtigkeits- und Fettgehalt der Kopfhaut zu normalisieren.

Die US 2010/247563 A1 offenbart eine topische Hautpflegezusammensetzung, die Folgendes umfasst: (a) eine Kombination von Extrakten aus Malpighia punicifolia, Argania spinosa, Myrciaria dubia, Punica granatum, Pinus sylvestris, Terminalia ferdinandiana, Linum usitatissimum, Ribes nigrum, Secale cereale, Algen und Hefe und (b) einen dermatologisch verträglichen Träger.

In der US 2018/092832 A1 ist eine organische Haarpflege zur Stimulierung des Haarwachstums und zur Reduzierung von Talg auf der Kopfhaut offenbart, die der Anwender alle 12 Stunden ohne die Unannehmlichkeiten von Ablagerungen, fettiger Kopfhaut oder Haarwaschen anwenden kann. Ein Ausspülen ist nicht erforderlich.

Die DE 10 2010 007736 A1 betrifft Zubereitungen zur Stabilisierung und zum Aufbau der Textur lebender Gewebe, insbesondere menschlicher Haut und ihrer Anhangsgebilde wie z.B. Haarfollikel und Nagelbett sowie Bindegewebe.

Das Platinum Stem Cell Elixir aus der Sarah Chapman Skinesis-Serie enthält kosmetische Wirkstoffe mit Pflanzenextrakten und ätherischen Ölen, um ein Anti-Aging-Ergebnis und ein Hautpflegeerlebnis zu erzielen.

Des weiteren sind im Stand der Technik zur Verwendung bei Haarausfall Öle bekannt, die auf die Kopfhaut aufgetragen werden. Nachteilig ist, dass solche Öle die Fettigkeit der Haare bzw. des Haaransatzes beträchtlich steigern. Ein fettiger Haaransatz ist aus ästhetischen Gründen häufig unerwünscht und hinterlässt Spuren auf Kissen, Kleidung etc., so dass nach der Applikation ein Auswaschen erforderlich ist. Ferner lassen sich Öle nur mühsam mit viel Shampoo auswaschen.

Mit einer konsequenten Anwendung des Produktes - und damit einer erfolgreichen Anwendung - kann nur gerechnet werden, wenn das Produkt physisch und psychisch gut angenommen wird und anwenderfreundlich ist. Synthetische Verbindungen sind aus ökologischen und gesundheitlichen Gründen zunehmend unerwünscht. Manche dieser synthetischen Verbindungen können allergische Reaktionen auslösen. Positiv wahrgenommen werden Produkte, die zu einem guten Volumen, guter Kämmbarkeit, Stärke und geringer Fettigkeit der Haare führen. Bevorzugt sind insbesondere solche Produkte, die die Fettigkeit des Haares bzw. des Haaransatzes nur unmerklich steigern und welche nicht ausgewaschen werden müssen.

Bekannt sind im Stand der Technik auch Produkte, bei denen eine ölhaltige Kapsel konsumiert wird. Dabei wirken die Inhaltsstoffe überwiegend systemisch und gelangen nicht direkt an den Wirkort. In vielen Fällen wird außerdem eine diskrete Einnahme, zum Beispiel in Form eines Getränkes an Stelle einer Kapsel, bevorzugt. Wichtig ist dabei, dass sich das Produkt gut mit Wasser verdünnen lässt. Insbesondere soll es bei Verdünnung mit Wasser nicht zur Koaleszenz von Fetttröpfchen kommen.

Ein weiterer Nachteil der Produkte im Stand der Technik besteht darin, dass diese darauf ausgelegt sind, entweder topisch auf der Kopfhaut oder oral appliziert zu werden. Somit können diese nicht zugleich von innen und außen wirken.

Zweiphasige Systeme wie Emulsionen stellen eine wichtige Darreichungsform dar, sowohl für Nahrungsergänzungsmittel als auch für kosmetische Präparate. Emulsionen sind komplexe zweiphasige Systeme aus zwei nicht miteinander mischbaren Phasen, von denen eine innere Phase in Tropfenform in einer äußeren Phase verteilt vorliegt. Häufig ist die äußere Phase eine wässrige Phase, die innere Phase wird durch ein Öl gebildet. Drei Arten der Stabilität bestimmen die langfristige Qualität und Lagereigenschaften von Emulsionen und anderen zweiphasigen Systemen: Die physikalische Stabilität, die chemische Stabilität und die mikrobiologische Stabilität.

Die physikalische Stabilität beschreibt das Vermögen einer Emulsion bzw. eines zweiphasigen Systems, die bei der Emulgierung durch mechanische Kräfte erreichte, feine und gleichmäßige Verteilung der dispersen Phase über einen längeren Zeitraum beizubehalten. Die feine und gleichmäßige Verteilung der dispersen Phase ist entscheidend für eine gleichmäßige Applikation des zweiphasigen Systems. Die physikalische Stabilität kann beispielsweise durch die Verwendung von Tensiden bzw. Emulgatoren, eine Angleichung der Dichte der beiden Phasen oder eine Erhöhung der Viskosität der äußeren Phase durch sogenannte Quasiemulgatoren, verbessert werden. Die Tenside werden in anionische, kationische, amphotere und nichtionische Tenside unterteilt, wobei die nichtionischen Tenside einige Vorteile aufweisen: Sie bilden im Wässrigen keine Ionen, reagieren neutral, sind nicht durch Elektrolyte beeinflussbar und indifferenter gegenüber chemischen Einflüssen.

Insbesondere im diätetischen und kosmetischen Bereich sind transparente oder zumindest durchscheinende Emulsionen und andere zweiphasige Systeme erwünscht. Dies kann in manchen Fällen durch eine innere Phase mit Tröpfchengrößen im Nanometerbereich erreicht werden. Solche Tröpfchengrößen werden beispielsweise durch Micellen, deren Bildung durch einen Emulgator und einen Coemulgator vermittelt werden kann, erreicht. Dabei werden als Emulgator häufig (nichtionische) ethoxylierte Tenside bzw. (nichtionische) synthetische Tenside mit hohem HLB-Wert und als Coemulgator Alkohole eingesetzt. Um Öle zu emulgieren, ist dabei oft eine erhebliche Menge an Emulgator und Coemulgator erforderlich. Allerdings werden synthetische Emulgatoren von einem Teil der Bevölkerung abgelehnt. Unbedenklicher sind natürlich vorkommende Emulgatoren, insbesondere bei oraler Aufnahme. Die Gesetzgebung erschwert zunehmend den Einsatz synthetischer Verbindungen in Kosmetikprodukten, was technisch anspruchsvolle Umgehungslösungen erforderlich macht. Die als Coemulgatoren eingesetzten Alkohole wie Ethanol und Propanol müssen insbesondere bei oralen Zubereitungen für Kinder, Alkoholiker, Schwangere etc. vermieden werden.

Neben der physikalischen Stabilität einer Emulsion bzw. eines zweiphasigen Systems muss auch die chemische und mikrobiologische Stabilität sichergestellt werden. Die chemische Stabilität ist in zweiphasigen Systemen insbesondere ein Problem, da Autoxidation und hydrolytische Vorgänge in Ölen und Fetten in der Anwesenheit von Wasser begünstigt sind. Ferner werden bei der Herstellung von Emulsionen häufig Luft und damit Sauerstoff eingearbeitet, welcher ebenfalls die Oxidation begünstigt. Dies ist insofern problematisch, da beispielweise oxidierte Fettsäuren nicht mehr nur nicht für physiologische Zwecke zur Verfügung stehen, sondern bei der Oxidation gesundheitsschädliche und reizende Produkte entstehen.

Emulsionen und andere zweiphasige Systeme stellen als wasserhaltige Systeme gute Nährböden für Mikroorganismen dar. Daher werden solchen zweiphasigen Systemen üblicherweise Konservierungsmittel beigefügt. Allerdings können Tenside die Wirksamkeit der Konservierungsmittel beeinträchtigen. Da die Konservierungsmittel in der Regel lipophil sind, gehen diese außerdem teilweise in die lipophile Phase über. Damit die Konzentration an Konservierungsmittel in der wässrigen Phase ausreichend hoch ist, um eine Konservierung zu bewirken, weisen solche zweiphasigen Systeme einen hohen Gehalt an Konservierungsmitteln auf. Allerdings können bestimmte Konservierungsmittel, beispielsweise die p-Hydroxybenzoesäureester, Unverträglichkeiten und Allergien auslösen.

Zunehmend erwünscht sind Produkte auf Basis verträglicher und natürlich vorkommender Inhalts- bzw. Hilfsstoffe, obgleich dies oft technisch schwer zu erreichen ist. Gerade Emulsionen und ähnliche zweiphasige Systeme stellen komplexe Systeme dar, in welchen Inhaltsstoffe nicht beliebig zusammengemischt werden können. Es muss vielmehr eine Mischung entwickelt werden, die den Bedürfnissen des Anwenders und den hohen technischen Anforderungen an die physikalische, chemische und mikrobiologische Stabilität gerecht wird.

Viele zweiphasige Systeme weisen das Problem auf, dass sie nicht tropffähig sind und sich nicht ohne nennenswerten Verlust durch eine Applikationsvorrichtung dosieren lassen. Verluste treten beispielsweise auf, wenn Teile des zweiphasigen Systems an Applikationsvorrichtungen wie Pipetten, Messbechern, Applikationsspitzen von Flaschen, Tropfbehältern oder Kunststoffspendern haften bleibt. Durch den Verlust sinkt die Menge verabreichter lipophiler Inhaltsstoffe in unvorhersehbarer Weise und ein Teil des wertvollen Produktes wird vergeudet.

Der vorliegenden Erfindung liegt als eine Aufgabe zu Grunde, mindestens ein Produkt zur Stärkung bzw. Förderung des Haarwuchses anzugeben.

Eine Aufgabe der vorliegenden Erfindung besteht darin, ein Produkt bereitzustellen, das bei topischer Applikation auf der Kopfhaut die Fettigkeit des Haares bzw. des Haaransatzes nur unmerklich steigert und welches nicht ausgewaschen werden muss. Eine Aufgabe der vorliegenden Erfindung besteht darin ein Produkt bereitzustellen, das vor allem bei topischer Applikation ein gutes Kopfhautgefühl und ein gutes Einziehen der Inhaltsstoffe in die Kopfhaut vermittelt, was entscheidend von der Zusammensetzung und Formulierung des Produktes beeinflusst wird.

Eine Aufgabe der Erfindung besteht darin, ein Produkt bereitzustellen, das zu einem guten Volumen, guter Kämmbarkeit und geringer Fettigkeit der Haare führt. Nur ein gutes Kopfhautgefühl führt zu einer konsequenten Anwendung des Produktes und damit zu einer erfolgreichen Anwendung.

Ferner besteht eine Aufgabe der Erfindung darin, ein Produkt bereitzustellen, das oral und diskret, zum Beispiel in Form einer Mischung in einem Getränk, eingenommen werden kann.

Ferner besteht eine Aufgabe der Erfindung darin, ein Produkt bereitzustellen, das von innen und von außen, systemisch und lokal wirken kann.

Eine weitere Aufgabe besteht darin, ein Produkt bereitzustellen, das bekömmlich ist, und vom Menschen psychologisch und physiologisch positiv aufgenommen wird.

Ferner soll das Produkt physikalisch, chemisch und mikrobiologisch stabil sein. Auf synthetische Emulgatoren und Konservierungsmittel soll soweit möglich verzichtet werden.

Ferner besteht eine Aufgabe der Erfindung darin, ein Produkt bereitzustellen, das zur leichten Applikation tropffähig ist bzw. gute Fließeigenschaften aufweist und sich ohne nennenswerten Verlust an lipophiler Phase durch eine Applikationsvorrichtung dosieren lässt.

Eine weitere Aufgabe der Erfindung liegt darin, die Verwendung eines Produktes als Nahrungsergänzungsmittel und/oder kosmetisches Produkt zur Stärkung bzw. Förderung des Haarwuchses anzugeben.

Ferner ist es eine Aufgabe der Erfindung, ein Verfahren zur Herstellung eines erfindungsgemäßen zweiphasigen Systems anzugeben.

### Gegenstand der Erfindung

Zumindest eine dieser Aufgaben wird durch ein zweiphasiges System nach Anspruch 1 und ein Kit nach Anspruch 12 gelöst. Mit Blick auf die Verwendung eines erfindungsgemäßen Produktes wird diese Aufgabe durch Anspruch 9, 11 und 13 gelöst. Mit Blick auf das Verfahren zur Herstellung eines erfindungsgemäßen zweiphasigen Systems wird diese Aufgabe durch Anspruch 14 gelöst.

Die Erfindung beruht auf dem Gedanken, ein zweiphasiges System anzugeben, das eine lipophile Phase und eine wässrige Phase umfasst, wobei das zweiphasige System 0,2 Gew.-% bis 3,0 Gew.-% Leinöl, 0,2 Gew.-% bis 3,0 Gew.-% Weizenkeimöl, 0,2 Gew.-% bis 3,0 Gew.-% Borretschöl, 0,001 Gew.-% bis 0,5 Gew.-% Roggenextrakt, 0,001 Gew.-% bis 0,5 Gew.-% Waldkiefernrindenextrakt und 0,1 Gew.-% bis 1,6 Gew.-% Sägepalmenöl enthält, bezogen auf das Gesamtgewicht des zweiphasigen Systems.

Die Erfinder der vorliegenden Patentanmeldung haben ein zweiphasiges System zur topischen Applikation auf der Kopfhaut und ein zweiphasiges System zur oralen Applikation sowie ein Kit, das ein zweiphasiges System zur topischen Applikation auf der Kopfhaut und ein zweiphasiges System zur oralen Applikation umfasst, entwickelt. Somit lassen sich Gemische mit Leinöl, Weizenkeimöl, Borretschöl, Roggenextrakt und Waldkiefernrindenextrakt unter Ausnutzung der unten beschriebenen Vorteile oral und/oder topisch auf der Kopfhaut applizieren.

Die natürlich vorkommenden Inhaltsstoffe dieser pflanzlichen Öle und Extrakte regen das Haarwachstum an, sowohl bei oraler als auch bei dermaler Aufnahme bzw. Applikation. Bevorzugt handelt es sich bei dem zweiphasigen System um ein zweiphasiges System zur Stärkung bzw. Förderung des Haarwuchses.

Durch eine feine Verteilung der lipophilen Phase in der wässrigen Phase kann die Bioverfügbarkeit der Inhaltsstoffe erhöht werden. Die Inhaltsstoffe können je nach Applikationsweg von innen und/oder von außen - systemisch und/oder lokal - wirken. Durch den Einsatz pflanzlicher Öle und Extrakte werden Nebenwirkungen vermieden und bestimmte Ausführungsformen des Produktes sind für Vegetarier geeignet.

Im Vergleich mit Ölen, die als einphasiges System, also als Öl, topisch auf der Kopfhaut appliziert werden, weist das erfindungsgemäße Produkt den Vorteil auf, dass weniger Fett aufgetragen wird und die Haare bzw. der Haaransatz und die Kopfhaut als weniger fettig empfunden wird. Ein weiterer Vorteil ist, dass das Produkt bei topischer Applikation auf der Kopfhaut nicht ausgewaschen werden muss.

Das zweiphasige System ist tropffähig und weist ein gutes Fließverhalten auf. Im Vergleich mit den meisten Ölen zeigt es eine geringe Viskosität. Es ist leicht aus Pipetten, Flaschen, Beuteln etc. applizierbar. Zweiphasige Systeme lassen sich so ohne nennenswerten Verlust durch eine Applikationsvorrichtung dosieren, da das zweiphasige System nicht an Applikationsspitzen, Pipetten, Messbechern, Flaschen, Beuteln etc. haften bleibt. Dadurch kann eine gleichbleibende Dosis an Lipiden verabreicht werden und weniger Produkt geht verloren. Die spezielle Zusammensetzung des zweiphasigen Systems vermittelt bei topischer Applikation auf der Kopfhaut ein gutes Kopfhautgefühl, ein gutes Einziehen in die Kopfhaut, ein gutes Haarvolumen und eine gute Kämmbarkeit der Haare.

Letztlich erlaubt das zweiphasige System die gleichzeitige Applikation hydrophiler bzw. wasserlöslicher (z.B. Biotin) und hydrophober bzw. wasserunlöslicher (z.B. Omega-6-Fettsäuren) Verbindungen. Ein erfindungsgemäßes zweiphasiges System kann hydrophile bzw. wasserlösliche und hydrophobe bzw. wasserunlösliche Substanzen an den Wirkort bringen.

Gegenüber Kapseln, die oral eingenommen werden, weist das zweiphasige System den Vorteil auf, dass es direkt oral aufgenommen oder in einem Getränk verdünnt werden kann. Somit kann die Einnahme diskret erfolgen. Kapseln werden auch aus Angst vor Prionen, einem vegetarischen oder veganen Lebensstil oder aus religiösen Gründen abgelehnt. Auch die direkte orale Einnahme öliger Substanzen wird häufig als unangenehm empfunden, was zu einer schlechten Compliance führt.

Der Ausdruck "zweiphasiges System" beschreibt ein zweiphasiges System, das eine wässrige Phase und eine lipophile Phase mit Lipiden umfasst. Zwischen wässriger und lipophiler Phase finden sich als Emulgator bevorzugt Lecithine. In bestimmten Ausführungsformen kann das System auch mehr als zwei Phasen aufweisen. In bestimmten Ausführungsformen stellt das zweiphasige System eine Emulsion dar. In bestimmten Ausführungsformen beschreibt der Begriff "zweiphasiges System" ein System mit einer wässrigen Phase und einer Wirkstoff/Emulgator-Phase. Die Wirkstoff/Emulgator-Phase kann Öltropfen, die mittels Emulgator stabilisiert sind, oder vorzugsweise Mizellen, in deren Struktur ein Wirkstoff eingebaut wird, umfassen.

Es bezeichnet Borretschöl Borretschsamenöl aus *Borago officinalis,* Leinöl Leinsamenöl aus *Linum usitatissimum,* Sägepalmenöl bzw. Sägepalmenfruchtöl aus *Serenoa serrulata, Serenoa repens.* Daneben sind dem Fachmann Weizenkeimöl *(Triticum vulgare),* Roggenextrakt *(Secale cereale)* und Waldkiefernrindenextrakt *(Pinus sylvestris)* und deren Gewinnung bekannt.

Ausführungsformen innerhalb dieser Patentanmeldung können beliebig miteinander kombiniert werden, sofern sich aus dem Gegenstand und der Beschreibung der Ausführungsformen nicht eindeutig Gegenteiliges ergibt.

Die Verben "enthalten" und "umfassen" und ihre Konjugationen enthalten auch das Verb "bestehen aus" mit seinen Konjugationen.

Besonders bevorzugte Ausführungsformen der Erfindung sind in den Unteransprüchen angegeben.

### Bevorzugte Ausführungsformen des zweiphasigen Systemes zur topischen Applikation auf der Kopfhaut und des zweiphasigen Systemes zur oralen Applikation

Das zweiphasige System enthält Leinöl, Weizenkeimöl, Borretschöl, Roggenextrakt, Waldkiefernrindenextrakt und Sägepalmenöl, in den Konzentrationen wie definiert in Anspruch 1.

Die natürlich vorkommenden Inhaltsstoffe von Leinöl, Weizenkeimöl, Borretschöl, Sägepalmenöl, Roggenextrakt und Waldkiefernrindenextrakt regen das Haarwachstum an.

In bevorzugten Ausführungsformen liegt die lipophile Phase in Mizellen, vorzugsweise mit einer mittleren Tröpfchengröße zwischen 5 nm und 500 nm, bevorzugter 10 nm und 250 nm, vor. In bestimmten Ausführungsformen liegt die lipophile Phase, bevorzugt zu mindestens 90 Gew.-% oder 95 Gew.-%, in mizellierter Form vor.

Die mittlere Tröpfchengröße wird bevorzugt durch Photonenkorrelationsspektroskopie (PCS) bestimmt. Durch Mizellen und eine kleine Tröpfchengröße kann eine besonders gute Bioverfügbarkeit und ein besonders transparentes zweiphasiges System erhalten werden.

In bevorzugten Ausführungsformen enthält das zweiphasige System 0,0001 Gew.-% bis 0,01 Gew.-% Biotin.

Ein Biotinmangel führt zu Haarausfall. Biotin ist in organischen Lösungsmittel nicht löslich, aber in Wasser. Vorteilhaft an dem zweiphasigen System ist, dass gemeinsam mit den fettlöslichen Wirkstoffen Biotin oral und topisch auf der Kopfhaut appliziert werden kann.

In bevorzugten Ausführungsformen ist das zweiphasige System, insbesondere bei Raumtemperatur, wassermischbar und/oder mit Wasser verdünnbar.

Vorteilhaft ist, dass das Produkt einfach verdünnt werden kann. Insbesondere tritt dabei keine Koaleszenz auf.

In einer Ausführungsform enthält das zweiphasige System keine p-Hydroxybenzoesäureester.

In einer Ausführungsform enthält das zweiphasige System (neben Lecithinen) keine weiteren Tenside, insbesondere keine ethoxylierten und/oder synthetischen Tenside.

In einer bevorzugten Ausführungsform bildet die wässrige Phase die äußere Phase.

### Weitere Ausführungsformen des zweiphasigen Systems zur topischen Applikation auf der Kopfhaut

In bevorzugten Ausführungsformen ist das zweiphasige System zur topischen Applikation, insbesondere zur topischen Applikation auf der Kopfhaut (des Menschen) geeignet.

Das zweiphasige System zur topischen Applikation auf der Kopfhaut enthält 0,2 Gew.-% bis 3,0 Gew.-% Leinöl, 0,2 Gew.-% bis 3,0 Gew.-% Weizenkeimöl, 0,2 Gew.-% bis 3,0 Gew.-% Borretschöl, 0,001 Gew.-% bis 0,5 Gew.-% Roggenextrakt, 0,001 Gew.-% bis 0,5 Gew.-% Waldkiefernrindenextrakt und 0,1 Gew.-% bis 1,6 Gew.-% Sägepalmenöl, Gew.-% jeweils bezogen auf das Gesamtgewicht des zweiphasigen Systems.

In einer Ausführungsform des zweiphasigen Systems zur topischen Applikation auf der Kopfhaut enthält das zweiphasige System 0,5 Gew.-% bis 1,4 Gew.-% Leinöl, 0,5 Gew.-% bis 1,4 Gew.-% Weizenkeimöl, 0,5 Gew.-% bis 1,4 Gew.-% Borretschöl, 0,01 Gew.-% bis 0,18 Gew.-% Roggenextrakt, 0,01 Gew.-% bis 0,18 Gew.-% Waldkiefernrindenextrakt und 0,2 Gew.-% bis 0,8 Gew.-% Sägepalmenöl, Gew.-% jeweils bezogen auf das Gesamtgewicht des zweiphasigen Systems.

In einer Ausführungsform des zweiphasigen Systems zur topischen Applikation auf der Kopfhaut enthält das zweiphasige System Peptide aus der Kurkuma (Curcuma longa).

In einer Ausführungsform des zweiphasigen Systems zur topischen Applikation auf der Kopfhaut enthält das zweiphasige System mehr als 50 Gew.-% oder 75 Gew.-% Wasser, bezogen auf das Gesamtgewicht des zweiphasigen Systems.

In einer Ausführungsform des zweiphasigen Systems zur topischen Applikation auf der Kopfhaut enthält das zweiphasige System 0,02 Gew.-% bis 1 Gew.-% Lecithine, bezogen auf das Gesamtgewicht des zweiphasigen Systems.

In einer Ausführungsform des zweiphasigen Systems zur topischen Applikation auf der Kopfhaut enthält das zweiphasige System 0,1 Gew.-% bis 2 Gew.-% Hydroxyacetophenone und 3 Gew.-% bis 14 Gew.-% Ethanol, Gew.-% jeweils bezogen auf das Gesamtgewicht des zweiphasigen Systems.

In einer Ausführungsform des zweiphasigen Systems zur topischen Applikation auf der Kopfhaut enthält das zweiphasige System 2 Gew.-% bis 12 Gew.-% Glycerin und/oder 0,01 Gew.-% bis 0,2 Gew.-% Gelbildner, insbesondere Gellan, Gew.-% jeweils bezogen auf das Gesamtgewicht des zweiphasigen Systems.

In einer besonders bevorzugten Ausführungsform des zweiphasigen Systems zur topischen Applikation auf der Kopfhaut enthält das zweiphasige System 0,2 Gew.-% bis 3,0 Gew.-% Leinöl, 0,2 Gew.-% bis 3,0 Gew.-% Weizenkeimöl, 0,2 Gew.-% bis 3,0 Gew.-% Borretschöl, 0,001 Gew.-% bis 0,5 Gew.-% Waldkiefernrindenextrakt, 0,001 Gew.-% bis 0,5 Gew.-% Roggenextrakt, 0,1 Gew.-% bis 1,6 Gew.-% Sägepalmenöl, 0,0001 Gew.-% bis 0,01 Gew.-% Biotin, 0,1 Gew.-% bis 2 Gew.-% Hydroxyacetophenone, 2 Gew.-% bis 12 Gew.-% Glycerin, 3 Gew.-% bis 14 Gew.-% Ethanol, 0,01 Gew.-% bis 0,2 Gew.-% Gellan, 0,02 Gew.-% bis 1 Gew.-% Lecithine, 0,01 Gew.-% bis 0,1 Gew.-% Tocopherol, 0,001 Gew.-% bis 0,02 Gew.-% Tetrahydropiperin, optional Peptide aus der Kurkuma (Curcuma longa) und (entionisiertes) Wasser ad 100 Gew.-%, Gew.-% jeweils bezogen auf das Gesamtgewicht des zweiphasigen Systems.

### Weitere Ausführungsformen des zweiphasigen Systemes zur oralen Applikation

In bestimmten Ausführungsformen ist das zweiphasige System zur oralen Applikation beim Menschen geeignet.

In einer Ausführungsform des zweiphasigen Systems zur oralen Applikation enthält das zweiphasige System Lecithine und 0,02 Gew.-% bis 8,02 Gew.-%, bevorzugt 0,02 Gew.-% bis 7,77 Gew.-%, besonders bevorzugt 0,02 Gew.-% bis 7,52 Gew.-%, Lipide und 45 Gew.-% bis 91 Gew.-%, bevorzugt 68 Gew.-% bis 91 Gew.-%, Glycerin, wobei die Summe an Gew.-% von Lecithinen und Lipiden an dem zweiphasigen System 0,5 Gew.-% bis 8,5 Gew.-%, bevorzugt 0,5 Gew.-% bis 8,25 Gew.-%, besonders bevorzugt 0,5 Gew.-% bis 8,0 Gew.-%, beträgt, Gew.-% jeweils bezogen auf das Gesamtgewicht des zweiphasigen Systems.

In einer Ausführungsform des zweiphasigen Systems zur oralen Applikation enthält das zweiphasige System 0,48 Gew.-% bis 8,46 Gew.-%, bevorzugt 0,48 Gew.-% bis 8,21 Gew.-%, besonders bevorzugt 0,48 Gew.-% bis 7,96 Gew.-%, Lecithine, Gew.-% jeweils bezogen auf das Gesamtgewicht des zweiphasigen Systems.

In diesem zweiphasigen System zur oralen Applikation verbleibt die lipophile Phase über einen langen Zeitraum fein und homogen dispergiert in der wässrigen Phase. Somit ist das erfindungsgemäße zweiphasige System in genannter Zusammensetzung physikalisch stabil. Dies ist insbesondere überraschend, da als Emulgatoren nur Lecithine eingesetzt werden und diese gegenüber der zu emulgierenden Substanz in geringen Mengen eingesetzt werden können. Beispielsweise kann die Masse an Lecithinen nur 5 % der Masse der zu emulgierenden Lipide betragen. Die physikalische Stabilität ist wichtig, um eine konstante Applikation der wirksamen Bestandteile sicherzustellen. Ein weiterer Vorteil dieses Systems besteht darin, dass Lecithine natürlichen Ursprungs sind und ohne Bedenken in größeren Mengen oral konsumiert werden können. Vorteilhaft ist ferner, dass einwertige Alkohole wie zum Beispiel Ethanol oder Propanol für die Stabilität des zweiphasigen Systems nicht erforderlich sind.

In einer Ausführungsform des zweiphasigen Systems zur oralen Applikation enthält das zweiphasige System keine synthetischen und/oder ethoxylierten Tenside.

In einer Ausführungsform des zweiphasigen Systems zur oralen Applikation enthält das zweiphasige System keine einwertigen Alkohole wie Ethanol oder Propanol und/oder keine zweiwertigen Alkohole.

Ein Vorteil ist, dass das erfindungsgemäße zweiphasige System zur oralen Applikation bei Verzicht auf einwertige Alkohole wie zum Beispiel Ethanol oder Propanol für Kinder, Alkoholiker, Schwangere etc. geeignet ist.

Überraschend ist, dass das Glycerin eine ausreichende Konservierung vermittelt und keine weiteren Konservierungsmittel erforderlich sind. Dadurch kann auf synthetische oder allergieauslösende Konservierungsmittel verzichtet werden. Möglicherweise können an Stelle des Glycerins oder zusätzlich zu dem Glycerin Kohlenhydrate wie Monosaccharide, Disaccharide, Maltit, Maltodextrin, und/oder Polyole mit mehr als zwei Alkoholgruppen wie z.B. Threit, Pentite, Hexite einzeln oder in Kombination eingesetzt werden.

Nur mittels natürlich vorkommender Inhaltsstoffe wird also ein stabiles zweiphasiges System zur oralen Applikation gebildet. Dadurch wird das zweiphasige System physiologisch und psychologisch besonders positiv angenommen und ist besonders gut verträglich. Das zweiphasige System weist ein geringes Risiko für Unverträglichkeiten wie Allergien auf. Überraschenderweise hat sich gezeigt, dass das zweiphasige System eine hohe Transparenz aufweist, woraus ein optisch attraktives Produkt resultiert. Die Transparenz resultiert zum einem aus dem geringen Massenanteil an Lecithinen und Lipiden. Vor allem aber weisen Glycerin und Triglyceride einen ähnlichen Brechungsindex auf. Indem die wässrige Phase des zweiphasigen Systems auf einen sehr hohen Gehalt an Glycerin eingestellt wurde, konnten die Erfinder den Brechungsindex der beiden Phasen angleichen, wodurch das zweiphasige System überraschenderweise transparent erscheint.

Überraschenderweise hat sich gezeigt, dass das zweiphasige System durch den geringen Massenanteil an Lecithinen und Lipiden tropffähig und damit leicht aus Pipetten, Applizierspitzen, Flaschen, Beuteln etc. applizierbar ist. Zweiphasige Systeme mit einem solch geringen Massenanteil an Lecithinen und Lipiden lassen sich ohne nennenswerten Verlust an lipophiler Phase durch eine Applikationsvorrichtung dosieren, da das zweiphasige System nicht an Pipetten, Applizierspitzen, Messbechern, Flaschen, Beuteln etc. haften bleibt. Dadurch kann eine gleichbleibende Dosis an Lipiden verabreicht werden und weniger Produkt geht bei der Anwendung verloren.

Insbesondere gelten als Lipide: Fettsäuren und deren Ester, insbesondere Triglyceride und Ethylester, inbesondere pflanzlichen, tierischen und/oder marinen Ursprungs; Omega-3-Fettsäuren wie z.B. Docosahexaensäure (DHA) und/oder Eicosapentaensäure (EPA) und deren Veresterungsprodukte; Omega-6-Fettsäuren und deren Veresterungsprodukte; die fettlöslichen Vitamine sowie Carotinoide, wie z.B. Lutein und Zeaxanthin; Vitamin A (Retinol) und dessen Derivate Retinal, Retinylpalmitat und Retinylacetat; Vitamin E als Sammelbegriff für Tocopherole und Tocotrienole (darunter fallen alpha-, beta-, gamma-, delta- Tocopherole und Tocotrienole, sowohl als natürliche Gemische als auch synthetisch in Reinform); Vitamin E-Derivate wie alpha-, beta-, gamma- und delta-Tocopherolacetat sowohl in optisch reiner Form als auch als Racemat; Vitamin D2 (Ergocalciferol), Vitamin D3 (Cholecalciferol), Vitamin K1 (Phyllochinon), Vitamin K2 (Menachinon), Vitamin K3 (Menadion) und Coenzym Q 10 H (Ubichinol) und Ubichinon-10; Perfluorcarbone.

Als Lipidquelle kommen insbesondere pflanzliche Rohstoffe wie Sojaöl, Sonnenblumenöl, Borretschöl, Leinöl, Weizenkeimöl, Sägepalmenöl, Roggenextrakt, Hanföl, insbesondere THC-freies Hanföl, Waldkiefernrindenextrakt, Algenöl und Orangenöl in Frage. Lipide in Form von Borretschöl (Borretschsamenöl; *Borago officinalis*), Leinöl (Leinsamenöl; *Linum usitatissimum*), Weizenkeimöl (*Triticum vulgare*), Sägepalmenöl (Sägepalmenfruchtöl; *Serenoa serrulata, Serenoa repens*), Roggenextrakt (*Secale cereale*) und Waldkiefernrindenextrakt (*Pinus sylvestris*) werden im erfindungsgemäßen zweiphasigen System verwendet.

Es werden insbesondere entölte Lecithinfraktionen eingesetzt. Dies bedeutet, dass die Lecithine im Wesentlichen frei von unpolaren Lipiden wie Fettsäuren und Triglyceriden sind. Der Gehalt an unpolaren Lipiden wie Fettsäuren und Triglyceriden beträgt bevorzugt weniger als 3 Gew.-%. Die Lecithine enthalten bevorzugt einen Anteil an polaren Lipiden (acetonunlöslicher Substanz) von 90 bis 100 Gew.-%. Die Lecithine umfassen entölte Fraktionen mit einem bevorzugten Gehalt an Phosphatidylcholin von 30 Gew.-% bis 100 Gew.-%.

Bei der Berechnung von Gew.-% werden im Zweifelsfall jene lipophilen Verbindungen, die einen Phosphat-/Phosphorsäurerest aufweisen, als Lecithine und nicht als Lipide angesehen, falls sich aus der Beschreibung nichts Anderes ergibt. Lipophile Substanzen, die keinen Phosphat-/Phosphorsäurerest aufweisen, werden im Zweifelsfall als Lipide angesehen, falls sich aus der Beschreibung nichts anderes ergibt.

Bevorzugt enthalten die Lecithine 40 Gew.-% bis 90 Gew.-% Phosphatidylcholin.

Die verwendeten Lecithine stammen bevorzugt aus Soja, Sonnenblume, Raps, Fisch, Milch und/oder Eiern, wobei nichttierische Quellen wie Soja, Sonnenblume und/oder Raps besonders bevorzugt sind.

In bestimmten Ausführungsformen enthält die wässrige Phase des zweiphasigen Systems zur oralen Applikation 50 Gew.-% bis 99 Gew.-%, bevorzugt 75 Gew.-% - 99 Gew.-%, Glycerin. Ein hoher Gehalt an Glycerin in der wässrigen Phase gewährt eine ausreichende Konservierung und eine Transparenz des zweiphasigen Systems zur oralen Applikation.

In bevorzugten Ausführungsformen beträgt in dem zweiphasigen System zur oralen Applikation das Massenverhältnis an Lecithinen zu Lipiden zwischen 8:1 und 1:20, besonders bevorzugt zwischen 1:1 und 1:12.

In bestimmten Ausführungsformen enthält das zweiphasige System zur oralen Applikation ein Algenöl, insbesondere ein Algenöl aus Ulkenia sp. und/oder Schizochytrium sp.

In einer bestimmten Ausführungsform enthält das zweiphasige System zur oralen Applikation Ubichinon-10 und/oder Ubichinol.

Ubichinon-10 und Ubichinol ist eine lipophile Verbindung und essentiell für die humane Atmungskette. Die reduzierte Form Ubichinol kann als Radikalfänger dienen. Das zweiphasige System zur oralen Applikation kann auch eine Versorgung mit Ubichinon-10 und Ubichinol sicherstellen.

In einer bestimmten Ausführungsform enthält das zweiphasige System Docosahexaensäure und Eicosapentaensäure.

Das zweiphasige System stellt eine effiziente Versorgung des Körpers mit Omega-3-Fettsäuren bereit.

In einer bestimmten bevorzugten Ausführungsform enthält das zweiphasige System keine Inhaltsstoffe tierischer Herkunft. Mit anderen Worten eignet sich das zweiphasige System für einen vegetarischen und/oder veganen Lebensstil. Vegetarier und vor allem Veganer leiden aufgrund ihres eingeschränkten Nahrungsmittelangebotes oft an einem Mangel an bestimmten Verbindungen und entsprechenden Mangelerscheinungen, was auch Haarausfall begünstigt. Vorteilhaft ist, dass diesen Menschen ein weiteres Produkt angeboten werden kann, um eine ausgewogenere Ernährung sicherzustellen.

In manchen Ausführungsformen des zweiphasigen Systems zur oralen Applikation besteht die wässrige Phase aus Wasser und Glycerin.

In manchen Ausführungsformen des zweiphasigen Systems zur oralen Applikation besteht die wässrige Phase im Wesentlichen, insbesondere zu > 90 Gew.-% oder zu > 95 Gew.-%, aus Wasser und Glycerin.

In bestimmten Ausführungsformen des zweiphasigen Systems zur oralen Applikation enthält die wässrige Phase 50 Gew.-% bis 1 Gew.-%, bevorzugt 25 Gew.-% bis 1 Gew.-%, Wasser.

In bestimmten Ausführungsformen des zweiphasigen Systems zur oralen Applikation enthält das zweiphasige System 50 Gew.-% bis 1 Gew.-%, bevorzugt 25 Gew.-% bis 1 Gew.-%, Wasser, bezogen auf das Gesamtgewicht des zweiphasigen Systems.

In einer bestimmten Ausführungsform des zweiphasigen Systems zur oralen Applikation enthält die wässrige Phase Monosaccharide, Disaccharide, Maltit, Maltodextrin, Threit, Pentite und/oder Hexite. Auch diese Verbindungen können die Wasseraktivität in der wässrigen Phase senken und zur Konservierung und Geschmacksbildung beitragen.

### Kit (Satz)

Die Erfindung beruht ferner auf dem Gedanken, ein Kit (bzw. einen Satz) anzugeben, das ein (erfindungsgemäßes) zweiphasiges System zur topischen Applikation auf der Kopfhaut und ein (erfindungsgemäßes) zweiphasiges System zur oralen Applikation umfasst.

Die Inhaltsstoffe können somit von innen und von außen - systemisch und lokal - wirken.

Bevorzugt ist das zweiphasige System zur topischen Applikation auf der Kopfhaut und das (erfindungsgemäße) zweiphasige System zur oralen Applikation in (separaten) Flaschen verpackt.

Zu weiteren Ausführungsformen, Vorteilen und Erläuterungen wird insbesondere auf die Ausführungen zu dem zweiphasigen System verwiesen.

### Verwendung

Bevorzugt erfolgt die Verwendung bzw. Applikation bzw. Anwendung beim Menschen. In bevorzugten Ausführungsformen ist die erfindungsgemäße Verwendung eine nichtmedizinische Verwendung.

Die Erfindung beruht auf dem Gedanken, die Verwendung eines erfindungsgemäßen zweiphasigen Systems zur topischen Applikation auf der Kopfhaut zur Stärkung des Haarwuchses anzugeben, wobei die Applikation des zweiphasigen Systems topisch auf der Kopfhaut erfolgt.

Vorteilhaft ist, dass nach der Applikation kein Auswaschen erforderlich ist. Ferner ist vorteilhaft, dass der Haaransatz nicht fettig wirkt/ist.

In bevorzugten Ausführungsformen wird ein zweiphasiges System zur topischen Applikation einmal täglich topisch auf der Kopfhaut appliziert.

Eine einmalige Applikation pro Tag genügt. Dies ist anwenderfreundlich und erhöht die Compliance.

In bevorzugten Ausführungsformen wird ein zweiphasiges System zur topischen Applikation topisch in Dosen von 2 mL bis 6 mL, bevorzugt von 3 mL bis 4 mL, pro Tag auf der Kopfhaut appliziert.

Die topische Applikation umfasst bevorzugt ein Einmassieren des zweiphasigen Systems in die Kopfhaut.

Die Erfindung beruht ferner auf dem Gedanken, die Verwendung eines erfindungsgemäßen zweiphasigen Systems zur oralen Applikation als Nahrungsergänzungsmittel und/oder kosmetisches Produkt, insbesondere zur Stärkung des Haarwuchses, anzugeben, wobei das zweiphasige System oral appliziert wird.

In bestimmten Ausführungsformen wird ein zweiphasiges System zur oralen Applikation einmal täglich oral appliziert.

Eine einmalige Applikation pro Tag genügt. Dies ist anwenderfreundlich und erhöht die Compliance.

In bestimmten Ausführungsformen wird ein zweiphasiges System zur oralen Applikation oral in Dosen von 3,5 mL bis 9 mL pro Tag appliziert.

Die orale Applikation kann ein Verdünnen des zweiphasigen Systems in einem Getränk umfassen.

Zu weiteren Ausführungsformen, Vorteilen und Erläuterungen wird insbesondere auf die Ausführungen zu dem zweiphasigen System bzw. zu dem Kit verwiesen.

Die Erfindung beruht ferner auf dem Gedanken, die Verwendung eines erfindungsgemäßen Kits als Nahrungsergänzungsmittel und/oder kosmetisches Produkt, insbesondere zur Stärkung des Haarwuchses, anzugeben.

Das (erfindungsgemäße) zweiphasige System zur topischen Applikation auf der Kopfhaut und das (erfindungsgemäße) zweiphasige System zur oralen Applikation in dem Kit werden jeweils wie oben beschrieben verwendet. Besonders vorteilhaft ist die Kombination von oraler und topischer Applikation. Die natürlich vorkommenden Inhaltsstoffe der pflanzlichen Öle und Extrakte regen das Haarwachstum an, sowohl bei oraler Aufnahme als auch bei dermaler Aufnahme. So können die Inhaltsstoffe von innen und von außen - systemisch und lokal - wirken.

Zu weiteren Ausführungsformen, Vorteilen und Erläuterungen wird insbesondere auf die Ausführungen zu dem zweiphasigen System und zu deren Verwendung bzw. zu dem Kit verwiesen.

Die Erfindung beruht ferner auf dem Gedanken, ein Verfahren zur Herstellung eines erfindungsgemäßen zweiphasigen Systems anzugeben. Das Verfahren umfasst vorzugsweise die Schritte:
a) Rühren einer wässrigen Zusammensetzung, wobei die wässrige Zusammensetzung bevorzugt 0,2 Gew.-% bis 10 Gew.-% Lecithine und 50 Gew.-% bis 99 Gew.-% Glycerin enthält,
b) Zugeben mindestens dreier Bestandteile ausgewählt aus Leinöl, Weizenkeimöl, Borretschöl, Roggenextrakt, Waldkiefernrindenextrakt und Sägepalmenöl, um ein Gemisch zu erhalten, bevorzugt derart, dass in dem Gemisch
   - 0,02 Gew.-% bis 8,02 Gew.-%, bevorzugt 0,02 Gew.-% bis 7,77 Gew.-%, besonders bevorzugt 0,02 Gew.-% bis 7,52 Gew.-%, Lipide enthalten sind;
   - die Summe an Gew.-% von Lecithinen und Lipiden an dem Gemisch 0,5 Gew.-% bis 8,5 Gew.-%, bevorzugt 0,5 Gew.-% bis 8,25 Gew.-%, besonders bevorzugt 0,5 Gew.-% bis 8,0 Gew.-%, beträgt;
   - 45 Gew.-% bis 91 Gew.-% Glycerin enthalten sind;
c) Rühren des Gemisches aus b) und Homogenisieren unter Hochdruck, um ein zweiphasiges System zu erhalten,
optional d) Zugeben des zweiphasigen Systems aus c) in eine wässrige Lösung von Hydroxyacetophenonen und/oder Peptiden aus der Kurkuma (Curcuma longa) und/oder Glycerin und/oder Ethanol und/oder Gelbildner, insbesondere Gellan, und Rühren.

Vorzugsweise wird das Verfahren derart ausgeführt, dass in dem Gemisch von Schritt b) und c) 0,48 Gew.-% bis 8,46 Gew.-%, bevorzugt 0,48 Gew.-% bis 8,21 Gew.-%, besonders bevorzugt 0,48 Gew.-% bis 7,96 Gew.-%, Lecithine enthalten sind.

Die Verfahrenstemperatur wird bevorzugt den Lecithinen und den zu solubilisierenden Lipiden angepasst. Bei hydrierten Lecithinen sind Verarbeitungstemperaturen zwischen 40 °C und 80 °C bevorzugt, bei ungesättigten Lecithinen kann bei Raumtemperatur gearbeitet werden. Bevorzugt wird oberhalb des Schmelzpunktes der Lipide gearbeitet.

Vorzugsweise wird auch während Schritt b) gerührt.

In Schritt b) können Tocopherol und/oder Tetrahydropiperin und weitere lipophile Stoffe zugegeben werden.

Biotin kann in der wässrigen Zusammensetzung von Schritt a) oder der wässrigen Lösung von Schritt d) enthalten sein.

Ein Hochdruckhomogenisator in Schritt c) ergibt eine feinere Dispergierung der lipophilen Phase, was zu einer besseren Bioverfügbarkeit und Transparenz führt. Mit einem Hochdruckhomogenisator wird bevorzugt erreicht, dass die lipophile Phase, insbesondere zu mindestens 95 Gew.-%, in mizellierter Form vorliegt. Ferner wird bevorzugt erreicht, dass die lipophile Phase Tröpfchen, insbesondere Mizellen, mit einer mittleren Tröpfchengröße zwischen 5 nm und 500 nm, bevorzugt 10 nm und 250 nm, bildet. Die mittlere Tröpfchengröße wird bevorzugt per Photonenkorrelationsspektroskopie (PCS) bestimmt.

Bevorzugt wird das Verfahren, insbesondere das Rühren des Gemisches und das Homogenisieren unter Hochdruck (Schritt c), unter Inertgas durchgeführt.

Das Rühren in Schritt d) erfolgt bevorzugt mit einem Rotor-Stator-Mischer.

Insbesondere Schritt d) führt zu einem zweiphasigen System zur topischen Applikation auf der Kopfhaut.

Ein zweiphasiges System zur oralen Applikation wird insbesondere durch Schritt c) erhalten.

Zu weiteren Ausführungsformen, Vorteilen und Erläuterungen wird insbesondere auf die Ausführungen zu dem zweiphasigen System verwiesen.

Teil der Erfindung ist auch ein zweiphasiges System, hergestellt durch das erfindungsgemäße Verfahren.

Die Erfindung wird nachfolgend anhand eines Ausführungsbeispieles näher erläutert.

Dabei zeigt
- Fig. 1: die Kopfhaut eines Probanden vor (links) und nach 6 Monaten täglicher Anwendung des erfindungsgemäßen Kits (rechts);
- Fig. 2: die Kopfhaut eines weiteren Probanden vor (links) und nach 6 Monaten täglicher Anwendung des erfindungsgemäßen Kits (rechts);
- Fig. 3: die Kopfhaut eines weiteren Probanden vor (links) und nach 6 Monaten täglicher Anwendung des erfindungsgemäßen Kits (rechts);
- Fig. 4: die Kopfhaut eines weiteren Probanden vor (links) und nach 6 Monaten täglicher Anwendung des erfindungsgemäßen Kits (rechts);
- Fig. 5: die Kopfhaut eines weiteren Probanden vor (links) und nach 6 Monaten täglicher Anwendung des erfindungsgemäßen Kits (rechts);
- Fig. 6: die Kopfhaut eines weiteren Probanden vor (links) und nach 6 Monaten täglicher Anwendung des erfindungsgemäßen Kits (rechts);
- Fig. 7: die Kopfhaut eines weiteren Probanden vor (links) und nach 6 Monaten täglicher Anwendung des erfindungsgemäßen Kits (rechts);
- Fig. 8: die Kopfhaut eines weiteren Probanden vor (links) und nach 6 Monaten täglicher Anwendung des erfindungsgemäßen Kits (rechts);

Es wurde ein Kit mit zwei erfindungsgemäßen zweiphasigen Systemen hergestellt, Haartropfen in einer Flasche mit Messbecher und ein Kopfhautfluid in einer Flasche mit Applikationsspitze.

Die Haartropfen zur oralen Anwendung enthielten etwa 1,6 Gew.-% Leinöl, etwa 1,6 Gew.-% Weizenkeimöl, etwa 1,6 Gew.-% Borretschöl, etwa 0,1 Gew.-% Roggenextrakt, etwa 0,1 Gew.-% Waldkiefernrindenextrakt und etwa 1 Gew.-% Sägepalmenöl. Daneben enthielten die Haartropfen etwa 0,0005 Gew.-% Biotin. Das zweiphasige System enthielt circa 14 Gew.-% Wasser, etwa 6 Gew.-% Lipide (insbesondere aus den Ölen und Extrakten), etwa 78 Gew.-% Glycerin, etwa 1,5 Gew.-% Lecithine, wobei die Summe an Gew.-% von Lecithinen und Lipiden an dem zweiphasigen System etwa 7,5 Gew.-% betrug.

Das Kopfhautfluid zur topischen Anwendung enthielt etwa 0,8 Gew.-% Leinöl, etwa 0,8 Gew.-% Weizenkeimöl, etwa 0,8 Gew.-% Borretschöl, etwa 0,05 Gew.-% Roggenextrakt, etwa 0,05 Gew.-% Waldkiefernrindenextrakt und etwa 0,5 Gew.-% Sägepalmenöl. Das zweiphasige System enthielt circa 82 Gew.-% entionisiertes Wasser, etwa 3 Gew.-% Lipide (insbesondere aus den Ölen und Extrakten), etwa 6 Gew.-% Glycerin, etwa 0,2 Gew.-% Lecithine PC 70, wobei die Summe an Gew.-% von Lecithinen und Lipiden an dem zweiphasigen System etwa 3,2 Gew.-% betrug. Das Kopfhautfluid enthielt ferner etwa 0,5 Gew.-% Hydroxyacetophenone, etwa 0,05 Gew.-% Gellan, etwa 7 Gew.-% Ethanol, etwa 1 Gew.-% Auszug mit Peptiden aus der Kurkuma, etwa 0,001 Gew.-% Biotin, etwa 0,0035 Gew.-% Tetrahydropiperin und etwa 0,025 Gew.-% Tocopherol.

Die Haartropfen wurden nach folgendem Herstellungsverfahren hergestellt:
Eine Lecithinfraktion aus Sonnenblumen mit einem Gehalt an Phosphatidylcholin von 70 Gew.-% wurde in einer 80 %-igen (Gew.-%), wässrigen Glycerinlösung unter Rühren vollständig dispergiert. Dieser Dispersion werden erst Biotin und dann Leinöl, Weizenkeimöl, Borretschöl, Roggenextrakt, Waldkiefernrindenextrakt und Sägepalmenöl, zugegeben und bei 50 °C zur Homogenität gerührt. Abschließend erfolgte eine Homogenisation mittels Hochdruckhomogenisator. Das Ergebnis war eine transparente, niedrigviskose, Dispersion. Die lipophile Phase bildete Tröpfchen mit einer Tröpfchengröße zwischen 5 nm und 500 nm.

Das Haarfluid wurde nach folgendem Herstellungsverfahren hergestellt:
Eine Lecithinfraktion aus Sonnenblumen mit einem Gehalt an Phosphatidylcholin von 70 Gew.-% wurde in einer 80 %-igen (Gew.-%), wässrigen Glycerinlösung unter Rühren vollständig dispergiert. Dieser Dispersion wurden Leinöl, Weizenkeimöl, Borretschöl, Roggenextrakt, Waldkiefernrindenextrakt Sägepalmenöl, Tetrahydropiperin und Tocopherol zugegeben und bei 50 °C zur Homogenität gerührt. Abschließend erfolgte eine Homogenisation mittels Hochdruckhomogenisator. Das erhaltene Homogenisat wurde in eine wässrige Lösung von Hydroxyacetophenon, Glycerin, Gellan, Ethanol, Auszug mit Peptiden aus der Kurkuma und Biotin eingearbeitet. Hierfür wurde ein Rotor-Stator-Mischer verwendet. Die lipophile Phase bildete Tröpfchen mit einer Tröpfchengröße zwischen 5 nm und 500 nm.

Das Kombinationsprodukt aus den Haartropfen (erfindungsgemäßes zweiphasiges System zur oralen Applikation) und dem Kopfhautfluid (erfindungsgemäßes zweiphasiges System zur topischen Applikation) wurde an zwölf Probanden getestet.

Einmal pro Tag verdünnte jeder Proband circa 8,5 mL der Haartropfen in ca. 150 mL Kaltgetränk (Raumtemperatur) und konsumierte das Getränk. Einmal pro Tag applizierte jeder Proband circa 3,3 mL Kopfhautfluid mit der Applikationsspitze der Flasche mit dem zweiphasigen System direkt auf die betroffenen Stellen der Kopfhaut. Das Kopfhautfluid wurde leicht einmassiert.

Das Kopfhautfluid führte nicht zu fettigem Haar und musste nicht ausgewaschen werden.

Die Haartropfen ließen sich bei Raumtemperatur in einem Getränk verdünnen. Es kam zu keiner Koaleszenz in dem Getränk.

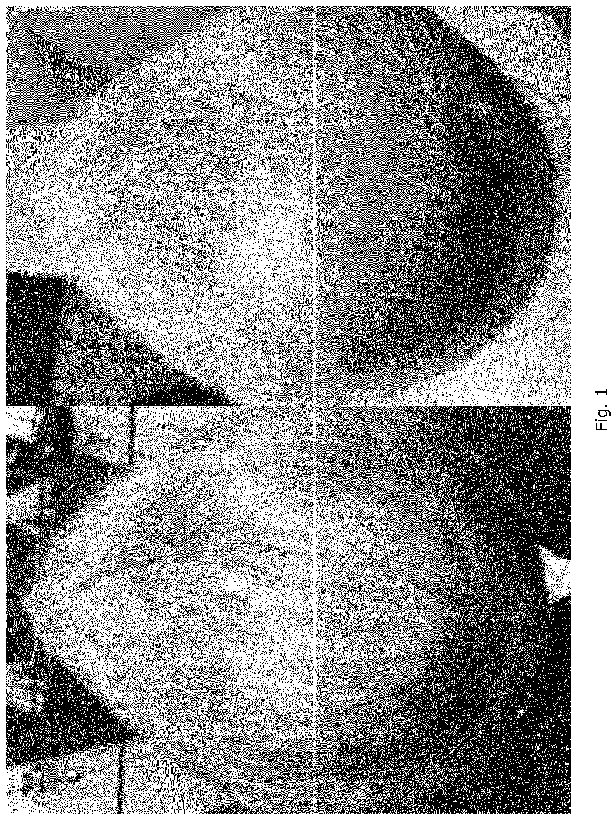

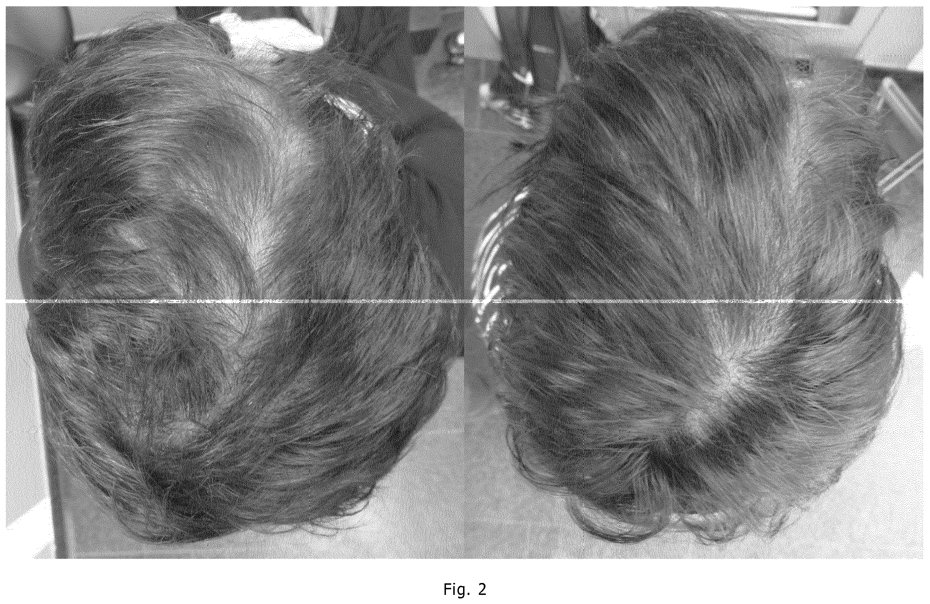

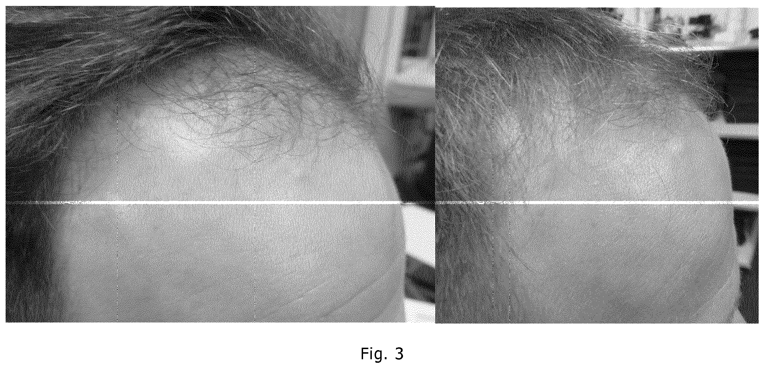

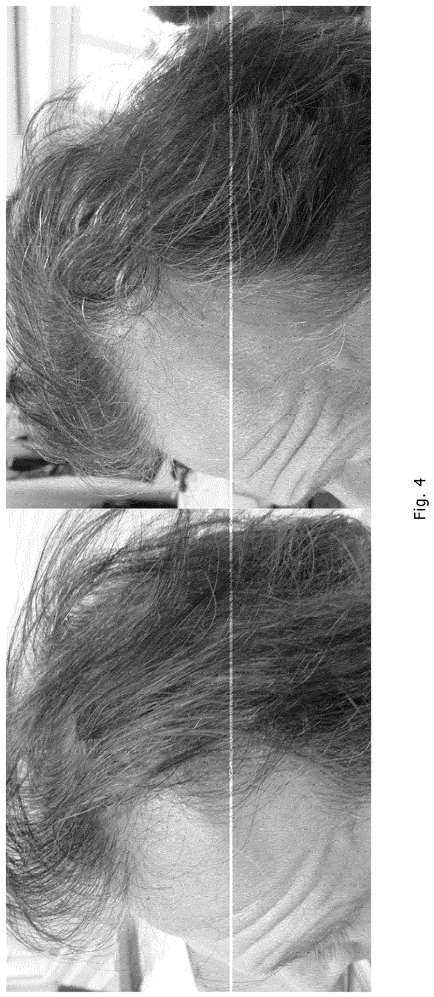

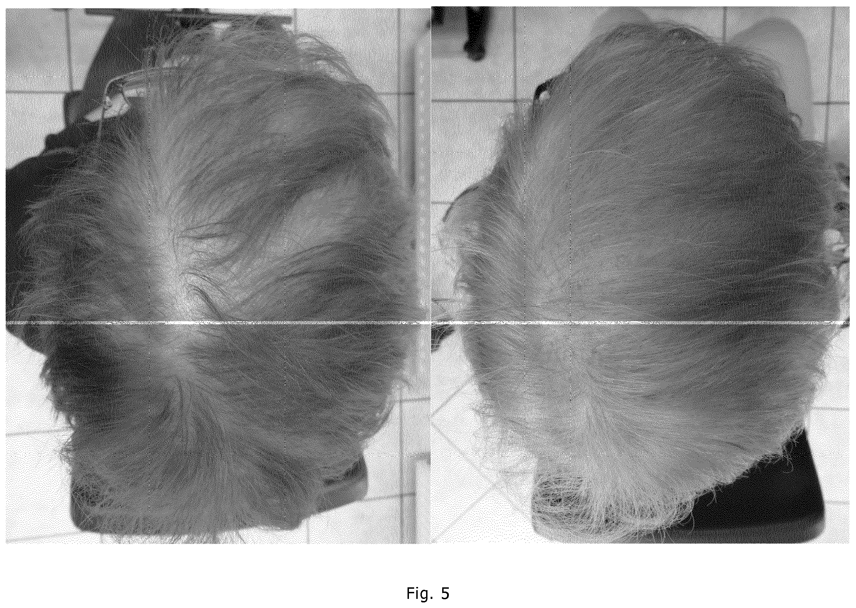

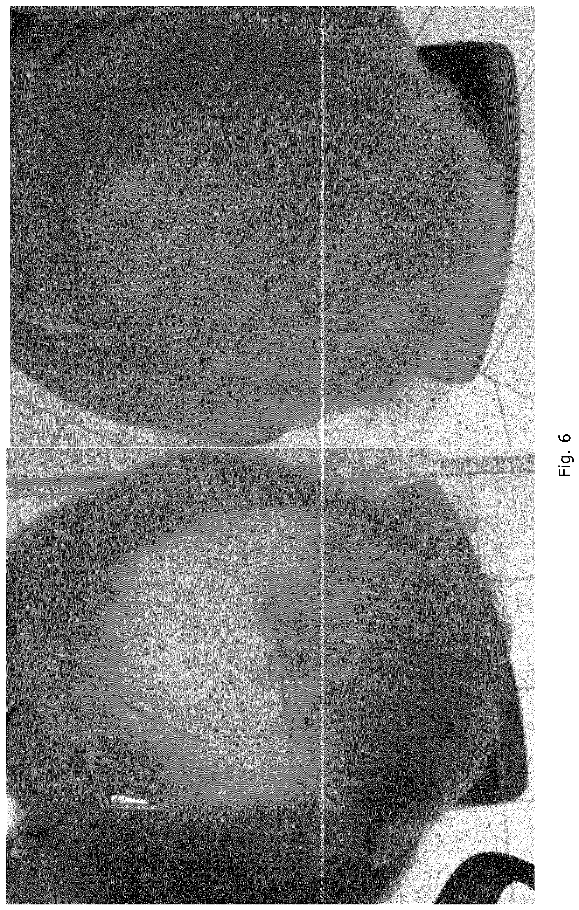

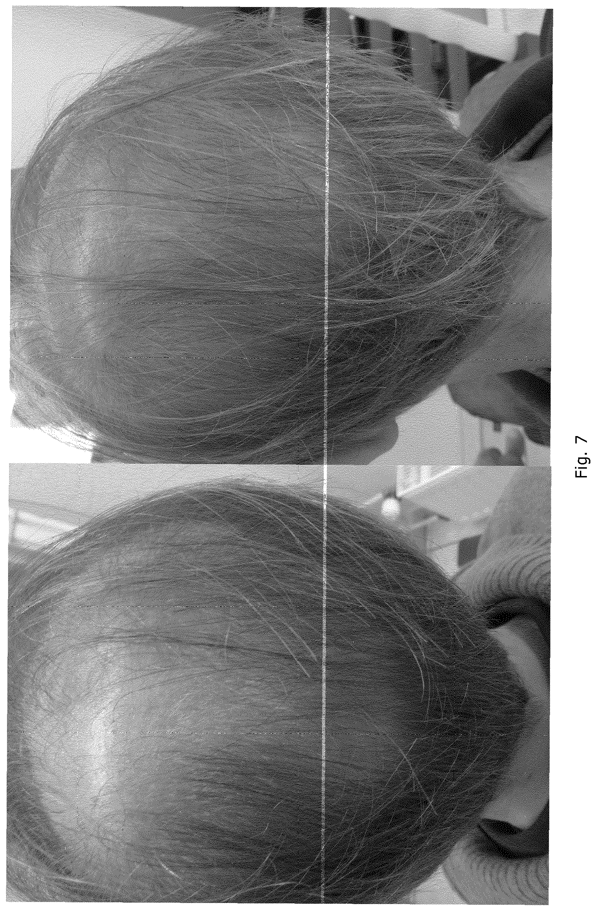

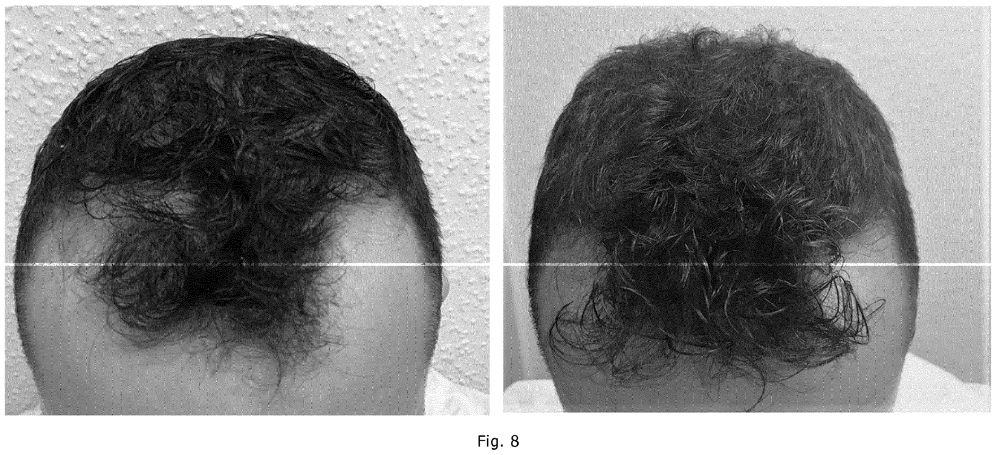

Acht der zwölf Probanden stellten nach 6 Monaten ein verstärktes Haarwachstum (vgl. Figuren 1 bis 8) fest und waren über das Ergebnis sehr erfreut.

Darüber hinaus wurde das Produkt von den Probanden folgendermaßen bewertet:

| Zweiphasiges System zur oralen Applikation | |
|---|---|
| Wie beurteilen Sie die Verträglichkeit? | sehr gut |
| Wie bewerten Sie die Verdünnbarkeit der Tropfen im Wasser? | gut |

| Zweiphasiges System zur topischen Applikation | |
|---|---|
| Wie empfinden Sie das Kopfhautgefühl nach dem Auftragen? | gut |
| Wie beurteilen Sie das Einziehen in die Kopfhaut? | gut |
| Wie bewerten Sie die Anwendung des Fluids? | gut |
| Wie empfinden Sie die Kämmbarkeit Ihrer Haare? | gut |
| Wie bewerten Sie die Verbesserung Ihres fettigen Haaransatzes? | gut |

| Resultate orale & topische Applikation | |
|---|---|
| Wie beurteilen Sie die Verwendung natürlicher Inhaltsstoffe? | sehr gut |

Dieses Beispiel beschreibt ein Kombinationspräparat (Kit) aus Haartropfen (erfindungsgemäßes zweiphasiges System zur oralen Applikation) und Kopfhautfluid (erfindungsgemäßes zweiphasiges System zur topischen Applikation). Auch die alleinige Verwendung der Haartropfen (erfindungsgemäßes zweiphasiges System) oder des Kopfhautfluids (erfindungsgemäßes zweiphasiges System) fördert das Haarwachstum, allerdings in etwas geringerem Maße. Dennoch weist das erfindungsgemäße zweiphasige System zur oralen Applikation und das erfindungsgemäße zweiphasige System zur topischen Applikation auf der Kopfhaut gegenüber dem Stand der Technik die oben beschriebenen und gezeigten Vorteile auf.

## Patentansprüche

1. Zweiphasiges System, umfassend eine lipophile Phase und eine wässrige Phase, **dadurch gekennzeichnet, dass**
das zweiphasige System 0,2 Gew.-% bis 3,0 Gew.-% Leinöl, 0,2 Gew.-% bis 3,0 Gew.-% Weizenkeimöl, 0,2 Gew.-% bis 3,0 Gew.-% Borretschöl, 0,001 Gew.-% bis 0,5 Gew.-% Roggenextrakt, 0,001 Gew.-% bis 0,5 Gew.-% Waldkiefernrindenextrakt und 0,1 Gew.-% bis 1,6 Gew.-% Sägepalmenöl enthält, bezogen auf das Gesamtgewicht des zweiphasigen Systems.

2. Zweiphasiges System nach Anspruch 1,
**dadurch gekennzeichnet, dass**
das zweiphasige System keine ethoxylierten und/oder synthetischen Tenside enthält.

3. Zweiphasiges System nach Anspruch 1 oder 2,
**dadurch gekennzeichnet, dass**
die lipophile Phase in Mizellen, vorzugsweise mit einer mittleren Tröpfchengröße zwischen 5 nm und 500 nm, bevorzugter 10 nm und 250 nm, vorliegt.

4. Zweiphasiges System nach einem der vorherigen Ansprüche, **dadurch gekennzeichnet, dass**
das zweiphasige System 0,0001 Gew.-% bis 0,01 Gew.-% Biotin enthält.

5. Zweiphasiges System nach einem der vorherigen Ansprüche,
**dadurch gekennzeichnet, dass**
das zweiphasige System, insbesondere bei Raumtemperatur, mit Wasser verdünnbar ist.

6. Zweiphasiges System nach einem der vorherigen Ansprüche,
**dadurch gekennzeichnet, dass**
das zweiphasige System 0,2 Gew.-% bis 0,8 Gew.-% Sägepalmenöl enthält, bezogen auf das Gesamtgewicht des zweiphasigen Systems.

7. Zweiphasiges System nach einem der vorherigen Ansprüche,
**dadurch gekennzeichnet, dass**
das zweiphasige System Peptide aus der Kurkuma (Curcuma longa) enthält.

8. Zweiphasiges System nach einem der vorherigen Ansprüche,
**dadurch gekennzeichnet, dass**
das zweiphasige System
0,2 Gew.-% bis 3,0 Gew.-% Leinöl,
0,2 Gew.-% bis 3,0 Gew.-% Weizenkeimöl,
0,2 Gew.-% bis 3,0 Gew.-% Borretschöl,
0,001 Gew.-% bis 0,5 Gew.-% Waldkiefernrindenextrakt,
0,001 Gew.-% bis 0,5 Gew.-% Roggenextrakt,
0,1 Gew.-% bis 1,6 Gew.-% Sägepalmenöl,
0,0001 Gew.-% bis 0,01 Gew.-% Biotin,
0,1 Gew.-% bis 2 Gew.-% Hydroxyacetophenone,
2 Gew.-% bis 12 Gew.-% Glycerin,
3 Gew.-% bis 14 Gew.-% Ethanol,
0,01 Gew.-% bis 0,2 Gew.-% Gellan,
0,02 Gew.-% bis 1 Gew.-% Lecithine,
0,01 Gew.-% bis 0,1 Gew.-% Tocopherol,
0,001 Gew.-% bis 0,02 Gew.-% Tetrahydropiperin,
optional Peptide aus der Kurkuma (Curcuma longa)
und (entionisiertes) Wasser ad 100 Gew.-%
enthält, bezogen auf das Gesamtgewicht des zweiphasigen Systems.

9. Verwendung eines zweiphasigen Systems nach einem der Ansprüche 1 bis 8 zur Stärkung des Haarwuchses, wobei die Applikation des zweiphasigen Systems topisch auf der Kopfhaut erfolgt.

10. Verwendung nach Anspruch 9, wobei nach der Applikation kein Auswaschen erforderlich ist und/oder der Haaransatz nicht fettig wirkt.

11. Verwendung eines zweiphasigen Systems als Nahrungsergänzungsmittel und/oder kosmetisches Produkt zur Stärkung des Haarwuchses, wobei das zweiphasige System eine lipophile Phase und eine wässrige Phase umfasst, wobei das zweiphasige System Leinöl, Weizenkeimöl, Borretschöl, Roggenextrakt und Waldkiefernrindenextrakt enthält, wobei das zweiphasige System oral appliziert wird.

12. Kit, umfassend ein zweiphasiges System nach einem der Ansprüche 1 bis 8 zur topischen Applikation auf der Kopfhaut und ein zweiphasiges System nach Anspruch 1 zur oralen Applikation.

13. Verwendung eines Kits nach Anspruch 12 als Nahrungsergänzungsmittel und/oder kosmetisches Produkt, insbesondere zur Stärkung des Haarwuchses.

14. Verfahren zur Herstellung eines zweiphasigen Systems nach einem der Ansprüche 1 bis 8, umfassend die Schritte:
a) Rühren einer wässrigen Zusammensetzung, wobei die wässrige Zusammensetzung bevorzugt 0,2 Gew.-% bis 10 Gew.-% Lecithine und 50 Gew.-% bis 99 Gew.-% Glycerin enthält,
b) Zugeben von Leinöl, Weizenkeimöl, Borretschöl, Roggenextrakt, Waldkiefernrindenextrakt und Sägepalmenöl, um ein Gemisch zu erhalten, bevorzugt derart, dass in dem Gemisch
- 0,02 Gew.-% bis 8,02 Gew.-%, bevorzugt 0,02 Gew.-% bis 7,77 Gew.-%, besonders bevorzugt 0,02 Gew.-% bis 7,52 Gew.-%, Lipide enthalten sind;
- die Summe an Gew.-% von Lecithinen und Lipiden an dem Gemisch 0,5 Gew.-% bis 8,5 Gew.-%, bevorzugt 0,5 Gew.-% bis 8,25 Gew.-%, besonders bevorzugt 0,5 Gew.-% bis 8,0 Gew.-%, beträgt;
- 45 Gew.-% bis 91 Gew.-% Glycerin enthalten sind;
c) Rühren des Gemisches aus b) und Homogenisieren unter Hochdruck, um ein zweiphasiges System zu erhalten,
d) optional Zugeben des zweiphasigen Systems aus c) in eine wässrige Lösung von Hydroxyacetophenonen und/oder Peptiden aus der Kurkuma (Curcuma longa) und/oder Glycerin und/oder Ethanol und/oder Gelbildner, insbesondere Gellan, und/oder Tocopherol und/oder Tetrahydropiperin.

## Claims

1. Two-phase system, comprising a lipophilic phase and an aqueous phase,
**characterized in that**
the two-phase system contains 0.2 wt.% to 3.0 wt.% linseed oil, 0.2 wt.% to 3.0 wt.% wheat germ oil, 0.2 wt.% to 3.0 wt.% borage oil, 0.001 wt.% to 0.5 wt.% rye extract, 0.001 wt.% to 0.5 wt.% Scotch pine bark extract, and 0.1 wt.% to 1.6 wt.% saw palmetto oil, in relation to the total weight of the two-phase system.

2. Two-phase system as claimed in claim 1,
**characterized in that**
the two-phase system does not contain ethoxylated and/or synthetic surfactants.

3. Two-phase system as claimed in claim 1 or 2,
**characterized in that**
the lipophilic phase is present in micelles, preferably having an average droplet size between 5 nm and 500 nm, more preferably 10 nm and 250 nm.

4. Two-phase system as claimed in any one of the preceding claims,
**characterized in that**
the two-phase system contains 0.0001 wt.% to 0.01 wt.% biotin.

5. Two-phase system as claimed in any one of the preceding claims,
**characterized in that**
the two-phase system can be diluted with water, in particular at room temperature.

6. Two-phase system as claimed in any one of the preceding claims,
**characterized in that**
the two-phase system contains 0.2 wt.% to 0.8 wt.% saw palmetto oil, in relation to the total weight of the two-phase system.

7. Two-phase system as claimed in any one of the preceding claims,
**characterized in that**
the two-phase system contains peptides from turmeric (Curcuma longa).

8. Two-phase system as claimed in any one of the preceding claims,
**characterized in that**
the two-phase system contains
0.2 wt.% to 3.0 wt.% linseed oil,
0.2 wt.% to 3.0 wt.% wheat germ oil,
0.2 wt.% to 3.0 wt.% borage oil,
0.001 wt.% to 0.5 wt.% Scotch pine bark extract,
0.001 wt.% to 0.5 wt.% rye extract,
0.1 wt.% to 1.6 wt.% saw palmetto oil,
0.0001 wt.% to 0.01 wt.% biotin,
0.1 wt.% to 2 wt.% hydroxyacetophenones,
2 wt.% to 12 wt.% glycerin,
3 wt.% to 14 wt.% ethanol,
0.01 wt.% to 0.2 wt.% gellan gum,
0.02 wt.% to 1 wt.% lecithins,
0.01 wt.% to 0.1 wt.% tocopherol,
0.001 wt.% to 0.02 wt.% tetrahydropiperine,
optionally peptides from turmeric (Curcuma longa),
and (deionized) water to make up 100 wt.%,
in relation to the total weight of the two-phase system.

9. Use of a two-phase system as claimed in any one of claims 1 to 8 for strengthening hair growth, wherein the two-phase system is applied topically to the scalp.

10. Use as claimed in claim 9, wherein no washing out is necessary after the application and/or the hairline does not appear greasy.

11. Use of a two-phase system as a nutritional supplement and/or cosmetic product for strengthening hair growth, wherein the two-phase system comprises a lipophilic phase and an aqueous phase, wherein the two-phase system contains linseed oil, wheat germ oil, borage oil, rye extract, and Scotch pine bark extract, wherein the two-phase system is applied orally.

12. Kit, comprising a two-phase system as claimed in any one of claims 1 to 8 for topical application to the scalp and a two-phase system as claimed in claim 1 for oral application.

13. Use of a kit as claimed in claim 12 as a nutritional supplement and/or cosmetic product, in particular for strengthening hair growth.

14. Method for producing a two-phase system as claimed in any one of claims 1 to 8, comprising the following steps:
a) stirring an aqueous composition, wherein aqueous composition preferably contains 0.2 wt.% to 10 wt.% lecithins and 50 wt.% to 99 wt.% glycerin,
b) adding linseed oil, wheat germ oil, borage oil, rye extract, Scotch pine bark extract, and saw palmetto oil to obtain a mixture, preferably such that the mixture contains
- 0.02 wt.% to 8.02 wt.%, preferably 0.02 wt.% to 7.77 wt.%, particularly preferably 0.02 wt.% to 7.52 wt.% lipids;
- the total of wt.% of lecithins and lipids in the mixture is 0.5 wt.% to 8.5 wt.%, preferably 0.5 wt.% to 8.25 wt.%, particularly preferably 0.5 wt.% to 8.0 wt.%;
- 45 wt.% to 91 wt.% glycerin;
c) stirring the mixture from b) and homogenizing it under high pressure to obtain a two-phase system,
d) optionally adding the two-phase system from c) to an aqueous solution of hydroxyacetophenones and/or peptides from turmeric (Curcuma longa) and/or glycerin and/or ethanol and/or gel formers, in particular gellan gum, and/or tocopherol and/or tetrahydropiperine.

## Revendications

1. Système diphasique, comprenant une phase lipophile et une phase aqueuse, **caractérisé en ce que** le système diphasique contient entre 0,2 % en poids et 3,0 % en poids d'huile de lin, entre 0,2 % en poids et 3,0 % en poids d'huile de germe de blé, entre 0,2 % en poids et 3,0 % en poids d'huile de bourrache, entre 0,001 % en poids et 0,5 % en poids d'extrait de seigle, entre 0,001 % en poids et 0,5 % en poids d'extrait d'écorce de pin sylvestre et entre 0,1 % en poids et 1,6 % en poids d'huile de palmier nain par rapport au poids total du système diphasique.

2. Système diphasique selon la revendication 1, **caractérisé en ce qu'**il ne contient pas de tensioactifs éthoxylés et/ou de synthèse.

3. Système diphasique selon la revendication 1 ou 2, **caractérisé en ce que** la phase lipophile se présente sous forme de micelles, ayant de préférence une grosseur moyenne de gouttelettes comprise entre 5 nm et 500 nm, de préférence entre 10 nm et 250 nm.

4. Système diphasique selon l'une des revendications précédentes, **caractérisé en ce que** le système diphasique contient entre 0,0001 % en poids et 0,01 % en poids de biotine.

5. Système diphasique selon l'une des revendications précédentes, **caractérisé en ce qu'**il peut être dilué avec de l'eau, en particulier à température ambiante.

6. Système diphasique selon l'une des revendications précédentes, **caractérisé en ce qu'**il contient entre 0,2 % en poids et 0,8 % en poids d'huile de palmier nain par rapport au poids total du système diphasique.

7. Système diphasique selon l'une des revendications précédentes, **caractérisé en ce qu'**il contient des peptides de curcuma (*Curcuma longa*).

8. Système diphasique selon l'une des revendications précédentes, **caractérisé en ce qu'**il contient
entre 0,2 % en poids et 3,0 % en poids d'huile de lin,
entre 0,2 % en poids et 3,0 % en poids d'huile de germe de blé,
entre 0,2 % en poids et 3,0 % en poids d'huile de bourrache,
entre 0,001 % en poids et 0,5 % en poids d'extrait d'écorce de pin sylvestre,
entre 0,001 % en poids et 0,5 % en poids d'extrait de seigle,
entre 0,1 % en poids et 1,6 % en poids d'huile de palmier nain,
entre 0,0001 % en poids et 0,01 % en poids de biotine,
entre 0,1 % en poids et 2 % en poids d'hydroxyacétophénones,
entre 2 % en poids et 12 % en poids de glycérine,
entre 3 % en poids et 14 % en poids d'éthanol,
entre 0,01 % en poids et 0,2 % en poids de gellane,
entre 0,02 % en poids et 1 % en poids de lécithines,
entre 0,01 % en poids et 0,1 % en poids de tocophérol,
entre 0,001 % en poids et 0,02 % en poids de tétrahydropipérine,
facultativement des peptides de curcuma (*Curcuma longa*),
et de l'eau (désionisée) q.s.p. 100 % en poids
par rapport au poids total du système diphasique.

9. Utilisation d'un système diphasique selon l'une des revendications 1 à 8 pour stimuler la pousse des cheveux, le système diphasique étant administré par voie topique sur le cuir chevelu.

10. Utilisation selon la revendication 9, dans laquelle aucun rinçage n'est nécessaire et/ou la racine des cheveux ne paraît pas grasse après l'administration.

11. Utilisation d'un système diphasique comme complément alimentaire et/ou comme produit cosmétique pour stimuler la pousse des cheveux, dans laquelle le système biphasique comprend une phase lipophile et une phase aqueuse, le système diphasique contenant de l'huile de lin, de l'huile de germe de blé, de l'huile de bourrache, de l'extrait de seigle et de l'extrait d'écorce de pin sylvestre, le système diphasique étant administré par voie orale.

12. Kit comprenant un système diphasique selon l'une des revendications 1 à 8 pour l'administration topique sur le cuir chevelu et un système diphasique selon la revendication 1 pour l'administration par voie orale.

13. Utilisation d'un kit selon la revendication 12 comme complément alimentaire et/ou comme produit cosmétique, en particulier pour stimuler la pousse des cheveux.

14. Procédé pour la fabrication d'un système diphasique selon l'une des revendications 1 à 8, comprenant les étapes suivantes :
a) agitation d'une composition aqueuse contenant de préférence entre 0,2 % en poids et 10 % en poids de lécithines et entre 50 % en poids et 99 % en poids de glycérine ;
b) ajout d'huile de lin, d'huile de germe de blé, d'huile de bourrache, d'extrait de seigle, d'extrait d'écorce de pin sylvestre et d'huile de palmier nain pour obtenir un mélange, de préférence de telle manière
- que le mélange contienne entre 0,02 % en poids et 8,02 % en poids, de préférence entre 0,02 % en poids et 7,77 % en poids, en particulier entre 0,02 % en poids et 7,52 % en poids, de lipides ;
- que la somme des pourcentages en poids des lécithines et des lipides dans le mélange représente entre 0,5 % en poids et 8,5 % en poids, de préférence entre 0,5 % en poids et 8,25 % en poids, en particulier entre 0,5 % en poids et 8,0 % en poids ;
- que le mélange contienne entre 45 % en poids et 91 % en poids de glycérine ;
c) agitation du mélange issu de b) et homogénéisation sous haute pression pour obtenir un système diphasique ;
d) facultativement, ajout du système diphasique issu de c) dans une solution aqueuse d'hydroxyacétophénones et/ou de peptides de curcuma (*Curcuma longa*) et/ou de glycérine et/ou d'éthanol et/ou de gélifiants, en particulier de gellane, et/ou de tocophérol et/ou de tétrahydropipérine.
